# EUROPEAN PATENT SPECIFICATION

(11) **EP 2 474 828 B1**
(45) Date of publication and mention of the grant of the patent: **08.08.2018**
(21) Application number: 10813909.8
(22) Date of filing: 30.08.2010
(51) Int. Cl.: G01N 33/50, G01N 33/53, G01N 21/84

(54) **DEVICE AND METHOD FOR QUANTITATIVELY MEASURING A SPECIMEN USING A CAMERA**
VORRICHTUNG UND VERFAHREN ZUR QUANTITATIVEN MESSUNG EINES PRÜFLINGS MIT EINER KAMERA
DISPOSITIF ET PROCÉDÉ DE MESURE QUANTITATIVE D'UN ÉCHANTILLON À L'AIDE D'UNE CAMÉRA

(30) Priority: 03.09.2009 KR 20090082869
(43) Date of publication of application: 11.07.2012
(73) Proprietor: OSANG HEALTHCARE CO., LTD., Anyang-si, Gyeonggi-do (KR)
(72) Inventor: BAE, Byeong-Woo, Anyang Kyunggi 431-080 (KR); Park, Cheol-Min, Anyang Kyunggi 431-080 (KR)
(74) Representative: D Young & Co LLP
(86) International application number: PCT/KR2010/005823
(87) International publication number: WO 2011/028000

(56) References cited:
- EP-A1- 0 646 784
- EP-A1- 1 391 728
- JP-A- 10 170 515
- KR-A- 20010 017 092
- KR-A- 20020 032 752
- KR-A- 20040 060 756
- US-A1- 2004 096 363
- US-A1- 2007 081 920
- US-A1- 2008 019 596
- US-B2- 7 344 081

## Description

### TECHNICAL FIELD

The present invention relates to a device, method, and system for quantitatively measuring an analyte, and more particularly, to a device, method, and system for quantitatively measuring an analyte by using a camera, which can read an identification code required to derive an accurate analysis result of an analyte while photographing an analyte reaction result by using a camera without additional equipment.

### BACKGROUND ART

In general, as interest in health increases, various technologies for physical health analysis using bio-samples are being introduced.

From thereamong, using diagnostic kits allows an extremely small amount of bio-analytes to be analyzed, thereby easily checking health conditions.

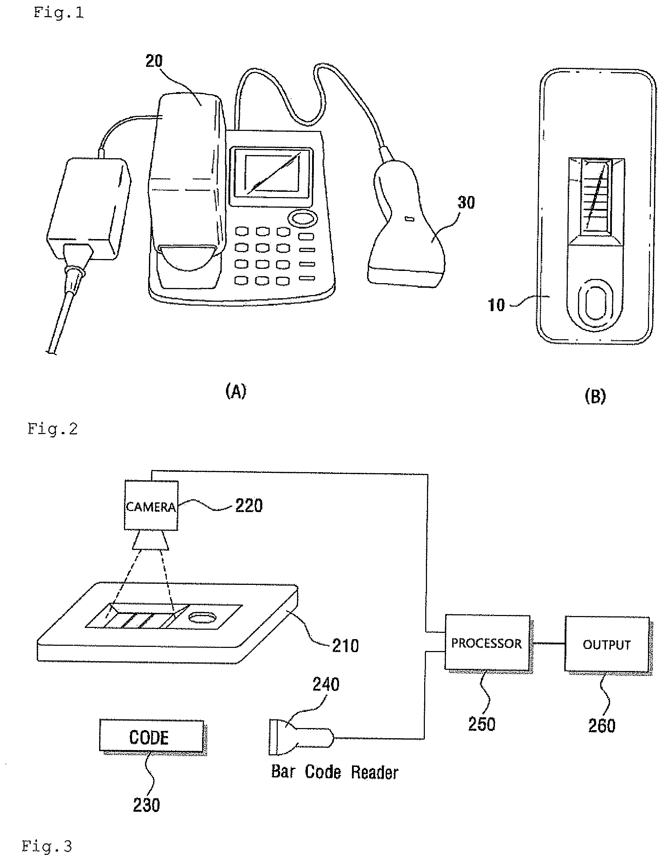

In this case, in order to analyze an end result as illustrated in Fig. 1, a result of an antigen-antibody reaction or enzyme reaction occurring in samples is provided through a diagnostic kit 10, and the result is photographed by a camera 20.

At this point, to obtain an accurate result, code information including effective information about respective diagnostic kits should be recognized.

To recognize this code information, according to existing methods, codes are implemented in bar codes, and the code information is recognized using a barcode reader separately from a camera.

Accordingly, as illustrated in Fig. 2, a reaction result is photographed using a camera 220, and a code 230 is read using a bar code reader 240 separately. In another method, codes may be recognized using RFID units.

However, using typical bar code readers or RFID units needs additional equipment for reading codes separately from photographing a reaction result of a diagnostic kit, and thus the miniaturizing of an analyzer is difficult and also additional cost arises.

Moreover, since additional operations of reading codes are needed, it takes more time for analysis and also operation processes are more complicated.

Other prior art includes EP 0,646,784 A1, US 7,344,081 B2 and US 2008/019596 A1.

### DISCLOSURE OF THE INVENTION

### TECHNICAL PROBLEM

The present invention provides a quantitative measurement device using a camera, which can obtain code information about an analyte kit as well as a sample reaction result through the photographing of images when analyzing the sample reaction result.

The present invention also provides an analyte kit, which can have an image photographable area to analyze the sample reaction result and the code information of the analyte kit through the photographing of images.

### TECHNICAL SOLUTION

The problem is solved by a quantitative measurement device according to claim 1 and corresponding independent method claim 6. A quantitative measurement device using a camera according to an example not forming part of the present invention includes a camera photographing a camera recognition area in one side of an analyte kit, wherein the camera recognition area comprises an analyte reaction result obtained by reaction of the analyte in blood and an identification code of the analyte kit; an image processing unit separating the images of the analyte reaction result and identification code from an image of the camera recognition code of the analyte kit photographed by the camera; a read unit reading the images of the photographed analyte reaction result and identification code, and a control unit allowing a result of reading the image of the analyte reaction result to be processed by using a result of reading the image of the identification code.

In this case, the read unit may include a first read unit reading the image of the analyte reaction result and a second read unit reading the image of the identification code.

And also, in order to process the result of reading the image of the analyte reaction result, the quantitative measurement device may further comprise a storage unit storing data corresponding to the identification code.

The result of reading the image of the analyte reaction result may be output through an output unit or display unit.

A quantitative measurement device using a camera according to an embodiment of the present invention includes a camera photographing a camera recognition area in one side of an analyte kit in the order of a first image and a second image, wherein the camera recognition area comprises an analyte reaction result obtained by reaction of the analyte in blood and an identification code of the analyte kit; a read unit reading the analyte reaction result and the identification code from the first image and the second image, respectively, which are photographed by the camera; and a control unit allowing a result of reading the image of the analyte reaction result to be processed by using a result of reading the image of the identification code.

The read unit extracts and reads the analyte reaction result and the identification code from a first virtual area and a second virtual area, respectively, of the camera recognition area in one side of the analyte kit.

The second virtual area may be a remaining area in the second image photographed by the camera, except the first virtual area.

The identification code may include at least one of a bar code, color pattern code, character, or number.

The identification code may include manufacturing lot information of the analyte kit, and the control unit may allow the result of reading the image of the analyte reaction result to be calibrated using the manufacturing lot information and allow the calibrated result to be output through a display unit.

The identification code may include an expire date of the analyte kit. In this case, the camera photographs the identification code before the analyte of blood is injected, and the control unit allows an error message to be output if the expire date of the analyte kit has passed.

The identification code may include analyte type information about types of analytes capable of being analyzed in the analyte kit. In this case, the camera photographs the identification code before the analyte of blood is injected, and the control unit allows an error message to be output if the analyte has the type which cannot be analyzed in the analyte kit.

An analyte kit used in a quantitative measurement device for quantitatively measuring an analyte in blood by using a camera according to an embodiment of the present invention includes a display window displaying an analyte reaction result obtained by reaction of the analyte in blood; and an identification code of the analyte kit, wherein the display window and the identification code are formed in a camera recognition area in one side of the analyte kit.

The analyte may be an antigen, and the analyte reaction result may be a result of an antigen-antibody reaction. The antigen may be Myoglobin, Creatine Kinase-MB(CK-MB), Troponin I, Pro-BNP, or D-dimer.

The antigen may be a tumor marker. For example, the tumor marker may be CA15-3 for metastatic breast cancer detection, CA19-9 for rectal cancer and pancreatic cancer detection, CA125 for ovarian cancer, CEA for rectal cancer, lung cancer, pancreatic cancer, gastrointestinal cancer, and breast cancer detection, PAS for prostate cancer detection, or AFP for liver cancer detection.

The camera recognition area may include a first virtual area and a second virtual area, the first virtual area including the display window and the second virtual area including the identification code.

The first virtual area and the second virtual area may be separated with a visible line.

A quantitative measurement method using a camera according to an example not forming part of the present invention includes the steps of: photographing a camera recognition area in one side of an analyte kit by the camera, wherein the camera recognition area comprises an analyte reaction result obtained by reaction of the analyte in blood and an identification code of the analyte kit; separating the images of the analyte reaction result and identification code from an image of the camera recognition code of the analyte kit photographed by the camera; reading the images of the analyte reaction result and identification code, and processing a result of reading the image of the analyte reaction result by using a result of reading the image of the identification code.

The processing may use the data corresponding to the identification code stored in the storage unit to process the result of reading the analyte reaction result.

A quantitative measurement method using a camera according to an embodiment of the present invention includes the first step of photographing a camera recognition area in one side of an analyte kit in the order of a first image and a second image by the camera, wherein the camera recognition area comprises an analyte reaction result obtained by reaction of the analyte in blood and an identification code of the analyte kit; the second step of reading the analyte reaction result from the first image; the third step of reading the identification code from the second image; and a fourth step of processing a result of reading the image of the analyte reaction result by using a result of reading the image of the identification code.

A quantitative measurement system using a camera according an embodiment of the present invention includes: an analyte kit including a display window displaying an analyte reaction result obtained by reaction of the analyte in blood, and an identification code included in a camera recognition area which is in the same side as the display window; and the quantitative measurement device according to the first or second embodiment.

### ADVANTAGEOUS EFFECTS

As described above, the device and method for quantitatively measuring an analyte by using a camera according to the present invention can recognize an analyte reaction result and code information by using one camera, thereby analyzing a sample quickly and miniaturizing the device because there is no need to include a code recognition device separately.

Furthermore, the device and method for quantitatively measuring an analyte by using a camera according to the present invention can recognize an analyte reaction result and code information by using one camera, thereby analyzing the recognized information with software.

### BRIEF DESCRIPTION OF THE DRAWINGS

Fig. 1 is a view of a system for quantitatively measuring an analyte in the related art.
Fig. 2 is a view illustrating a concept of the prior art.
Fig. 3 is a view illustrating a basic concept of the present invention.
Fig. 4 is a view illustrating an analyte kit according to a first embodiment of the present invention.
Fig. 5 is a view illustrating various methods of implementing identification codes according to the present invention.
Fig. 6 is a view illustrating an analyte kit according to a second embodiment of the present invention.
Fig. 7 is a view illustrating a configuration of a system for quantitatively measuring an analyte by using a camera according to the present invention.
Fig. 8 is a view illustrating a method of quantitatively measuring an analyte by using a camera according to a first embodiment of the present invention.
Fig. 9 is a view illustrating a method of quantitatively measuring an analyte by using a camera according to a second embodiment of the present invention.

### BEST MODE FOR INVENTION

Hereinafter, preferred embodiments will be described in more detail with reference to the accompanying drawings. Moreover, detailed descriptions related to well-known functions or configurations will be ruled out in order not to unnecessarily obscure subject matters of the present invention.

In the description of the present invention, a "analyte" term is uniformly used as an analyte in a sample taken from an organism and then used for analysis, and a "analyte kit" term is uniformly used as a diagnostic kit. And also, an "identification code" term is uniformly used as a code including code information. However, technical features are not limited to these terms.

Fig. 3 is a view illustrating a basic concept of the present invention.

As illustrated in Fig. 3, in order to analyze an analyte through a quantitative measurement device according to the present invention, an analyte reaction result and an identification code are in the same side of an analyte kit 300.

At this point, an analyte obtained from blood is provided to the analyte kit 300, moved by chromatography into a reaction area of the analyte kit 300, and reacted therein. The result of the reaction area is referred to as the analyte reaction result.

For example, blood is moved by chromatography into a reaction area and reacted therein, and thus the color of the reaction area changes according to a concentration of an analyte in the blood. At this point, the color may be the analyte reaction result.

As a specific example, the analyte may be an antigen, and the analyte reaction result may be a result of an antigen-antibody reaction. Herein, the antigen may be Myoglobin, Creatine Kinase-MB(CK-MB), Troponin I, Pro-BNP, or D-dimer.

And also, the antigen may be a tumor marker. For example, the tumor marker may be CA15-3 for metastatic breast cancer detection, CA19-9 for rectal cancer and pancreatic cancer detection, CA125 for ovarian cancer, CEA for rectal cancer, lung cancer, pancreatic cancer, gastrointestinal cancer, and breast cancer detection, PAS for prostate cancer detection, or AFP for liver cancer detection.

However, this is just one example, and thus many analytes may be analyzed and analyte reaction results thereof may be various.

And also, an identification code means information including a separate code value required to obtain effective information about an analyte reaction result.

For example, the identification code may be manufacturing lot information about the analyte kit 300. The manufacturing lot information about the analyte kit 300 may be used to calibrate a result of reading an analyte reaction result.

As another example, the identification code may be an expire date of the analyte kit 300. The identification code including the expire date is used to determine whether the analyte kit is to be used.

The identification code may be implemented in various forms, such as bar code, color pattern, number, and character.

The analyte kit 300 having an analyte reaction result and an identification code in one side thereof is photographed using a camera 310. An image photographed by the camera 310 is analyzed by a processor 320 and then the analysis result is output to an output unit 330.

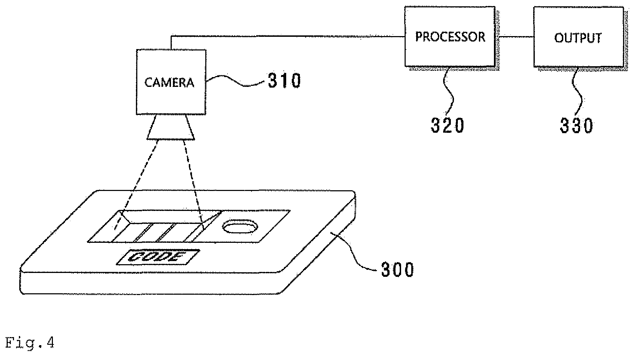

Fig. 4 is a view illustrating a first embodiment of an analyte kit according to the present invention.

As illustrated in FIG. 4, the analyte kit 300 includes an injection hole 330 for injecting an analyte, a display window 320 display an analysis result of the injected analyte, and an identification code 340.

In this case, the display window 320 indicates an reaction area where an analyte is reacted.

To obtain an analyte analysis result using a camera, the display window 320, reaction area, and identification code should be in the same side.

However since the camera reads the analyte reaction result and identification code separately, the display window 320 and the identification code 340 may be spaced apart by a certain interval.

And also, the identification code 340 exists in an area of the side of the analyte kit 300, which can be photographed by a camera.

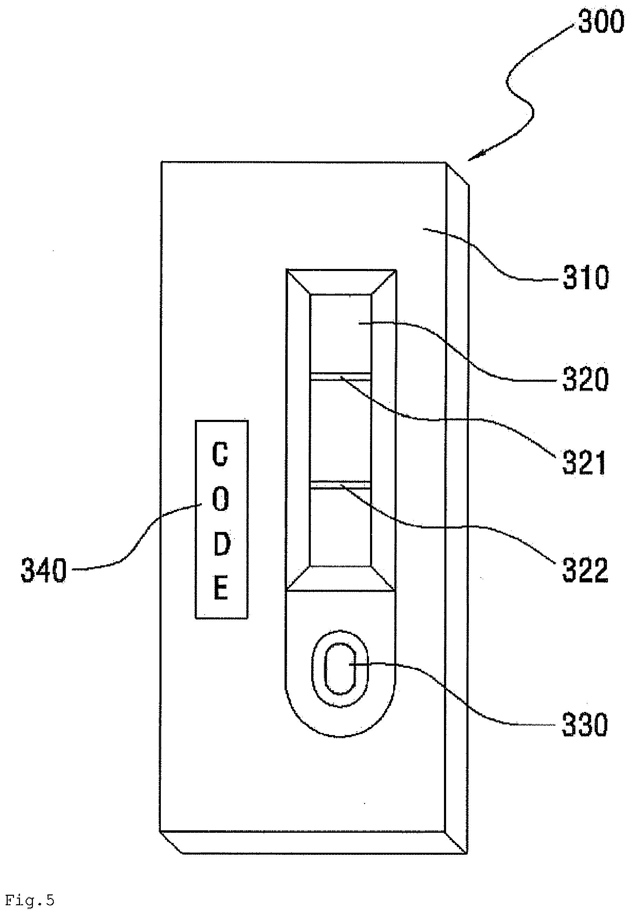

The identification code 340 may be implemented in various methods, as shown in Fig. 5.

For example, it may be implemented with a barcode such as 1-dimensional barcode or 2-dimensional barcode, color pattern, numbers, or characters.

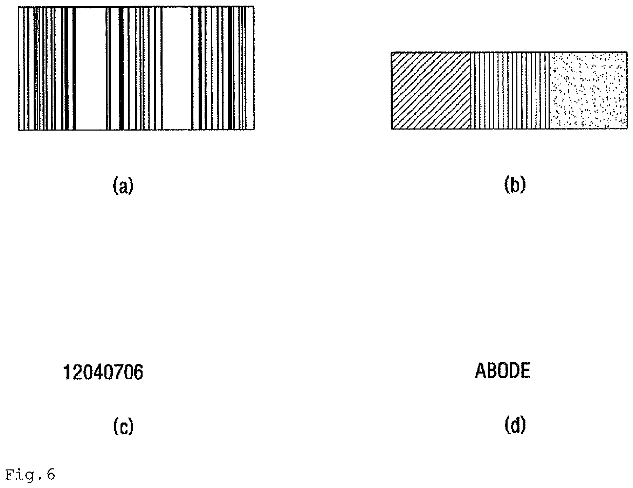

Fig. 6 is a view illustrating an analyte kit according to a second embodiment of the present invention.

As illustrated in Fig. 6(A), a virtual area 600 is shown, which can be recognized by a camera in the analyte kit 300 having the identification code 340 marked thereon.

Accordingly, the identification code 340 should be marked in the virtual area 600. Herein, the virtual area 600 may not be indicated. If necessary, the virtual area 600 may be indicated with a dotted line.

However, the virtual area 600 may be recognized in various methods by an algorithm during a process of reading an analyte reaction result or identification code.

In Fig. 6(B), a first virtual area 610 where an analyte reaction result of a display window 320 is photographed and a second virtual area 620 where an identification code is photographed are separated.

In this case, the analyte reaction result may be read from the first virtual area 610 of a photographed image, and the identification code may be read from the second virtual area 620 thereof. The second virtual area 620 may be a remaining area in the photographed image, except the first virtual area 610.

Herein, the first virtual area 610 and the second virtual area 620 may be recognized during a process by an algorithm even when there are no indications. And also, the areas may be indicated in a specific method.

Like this, the process of reading the analyte reaction result may be different from the process of reading the code information according to an area division method of the analyte kit 300.

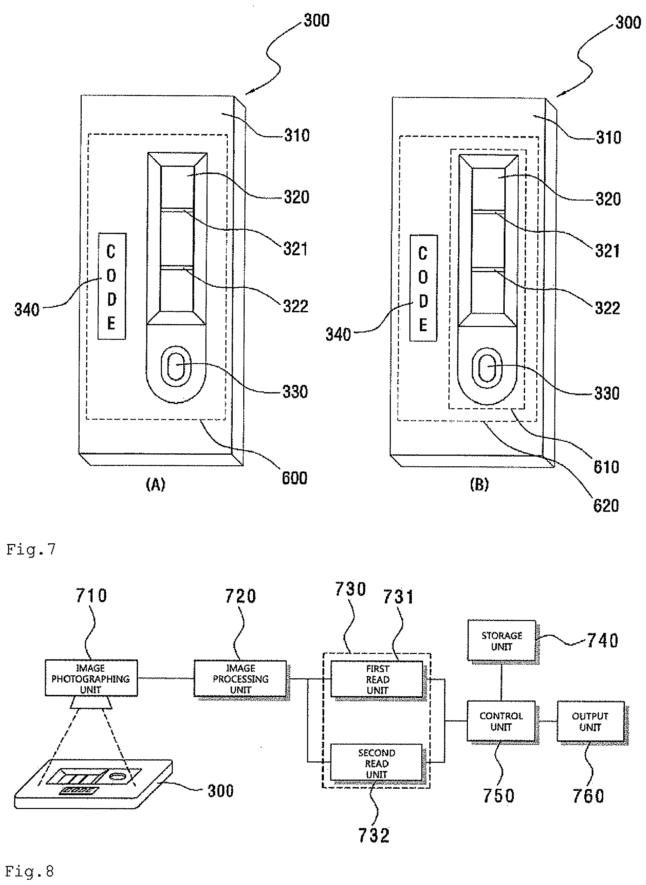

Fig. 7 is a view illustrating a configuration of a device for quantitatively measuring an analyte by using a camera according to the present invention.

As illustrated in FIG. 7, the quantitative measurement device includes an image photographing unit 710, an image processing unit 720, a read unit 730, a storage unit 740, a control unit 750, and a output unit 760. Herein, the image photographing unit 710 may be generally a camera.

The image photographing unit 710 photographs one side of the analyte kit 300 having an analyte reaction result and an identification code therein. In this case, the side of the analyte kit 300 is photographed at one time.

The image processing unit 720 separates an analyte reaction result image and an identification code image from the image of the side of the analyte kit 300 which is photographed by the image photographing unit 710.

The method of separating the analyte reaction result image and the identification code image may be implemented in various algorithms.

For example, the analyte reaction result image and the identification code image are separated according to their positions in a virtual area, as illustrated in Fig. 6(A). In a photographed image, a central portion may be recognized as the analyte reaction result image, and relatively an edge area may be recognized as the identification code area.

As another example, in a case where the analyte kit 300 has the first virtual area 610 and the second virtual area 620 as illustrated in Fig. 6(B), the first virtual area 610 is recognized as the analyte reaction result area, and the second virtual area 620 is recognized as the identification code area. The first virtual area 610 and the second virtual area 620 may be predetermined in the image processing unit 720.

The read unit 730 reads the analyte reaction result image and the identification code image, respectively.

The read unit 730 may have a first read unit 731 for reading the analyte reaction result and a second read unit 732 for reading the code information.

The control unit 750 allows the result of reading the analyte reaction result to be processed and then output by using the result of reading the identification code image. In this case, the control unit 750 may load information corresponding to the read identification code from the storage unit 740 storing information corresponding to identification codes and thus obtain effective information about the analyte.

In particular, when the identification code is manufacturing lot information about the analyte kit, the control unit 750 allows a result of reading the analyte reaction result to be calibrated using the manufacturing lot information which is read from the identification code.

When the identification code is an expire date, the control unit 750 allows an error message to be output through the output unit 760 when the expire date has passed according to a result of reading the identification code photographed by the image photographing unit 710.

And also, in a case where the identification code indicates a type of an analyte to be analyzed in an analyte kit, the control unit 750 allows an error message "unable to be analyzed" to be output through the output unit 760 when the analyte has the type which cannot be analyzed by the quantitative measurement device.

The output unit 760 outputs an effective end result for an analyte which is obtained by the control unit 750. Depending on the case, the output unit 760 may be referred to as various terms such as a display unit.

As another method, the image photographing unit 710 photographs one side of the analyte kit having an analyte reaction result and an identification code therein in the order of a first image and a second image.

In this case, the first image and the second image are the same or different with respect to a predetermined area.

The first image may be used for the analyte reaction result, and the second image may be used for the identification code.

The read unit 730 reads the analyte reaction result and the identification code from the first image and the second image, respectively, which are photographed by the image photographing unit 710.

In this case, the read unit 730 may extract and read the analyte reaction result and the identification code from the predetermined first image and second image, respectively.

The control unit 750 allows the result of reading the analyte reaction result to be processed and then output by using the result of reading the identification code image. In this case, the control unit 750 may load information corresponding to the read identification code from the storage unit 740 storing information corresponding to identification codes and thus obtain effective information about the analyte.

The output unit 760 outputs an effective end result for an analyte which is obtained by the control unit 750.

Fig. 8 is a view illustrating a method of quantitatively measuring an analyte by using a camera according to a first embodiment of the present invention.

As illustrated in Fig. 8, in the method of quantitatively measuring an analyte by using a camera according to a first embodiment of the present invention, one side of an analyte kit including an analyte reaction result and an identification code is photographed by an image photographing unit, i.e., a camera (S810).

After the side of the analyte kit is photographed, an analyte reaction result image included in a first area and an identification code image included in a second area are separated from the photographed image of the side of the analyte kit (S820). In this case, the first area and the second area may be predetermined.

After the analyte reaction result image and the identification code image are separated, the analyte reaction result of the first area is read (S830).

And also, the identification code of the second area is read (S840).

After the analyte reaction result and the identification code are read, a result of reading the analyte reaction result is analyzed using a result of reading the identification code, and then a reaction result is output (S850). In this case, analyzing the result of reading the analyte reaction result and then outputting the analysis result denotes loading information corresponding to the read identification code from a storage unit storing information corresponding to identification codes to obtain effective information about the analyte. After the effective information about the analyte is obtained, this information is output through an output unit (S860).

For example, in a case where the identification code includes manufacturing lot information about the analyte kit, a result of reading the analyte reaction result is calibrated using the manufacturing lot information, and a result of reading the calibrated analyte reaction result is output.

In a case where the identification code of the second area includes expire date information and is photographed by a camera before an analyte is injected, it is determined whether the expire date of the analyte kit included in the identification code has passed, and if the expire date has passed, an error message is output.

And also, in a case where the identification code includes analyte type information about types of analytes capable of being analyzed in the analyte kit and is photographed by a camera before the analyte is injected, if the analyte has the type which cannot be analyzed, an error message is output.

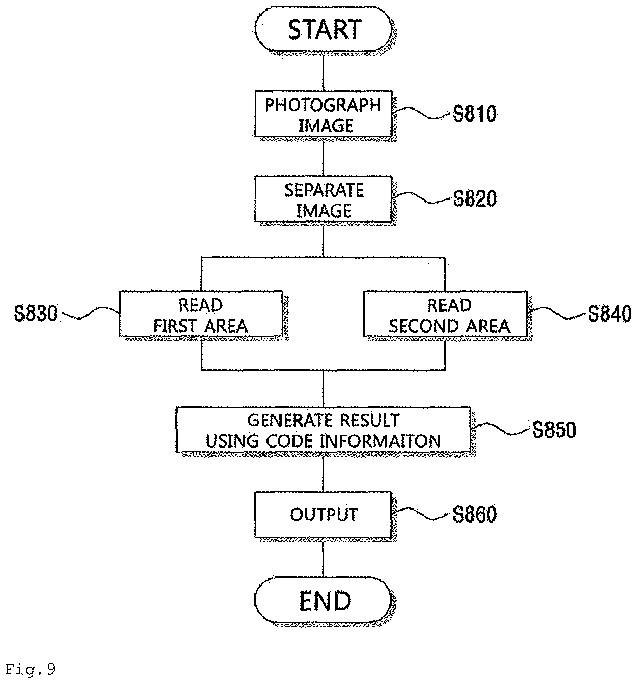

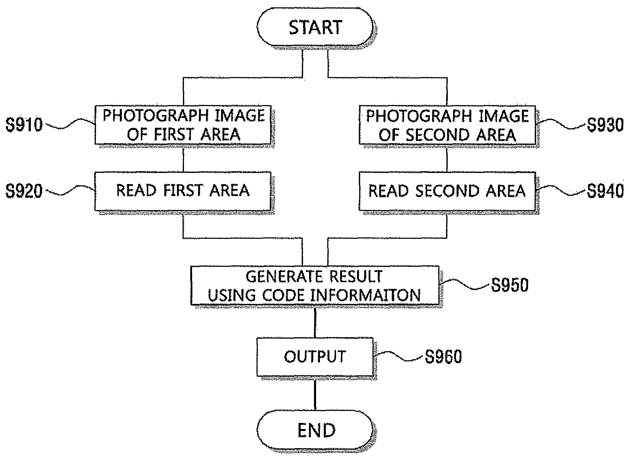

Fig. 9 is a view illustrating a method of quantitatively measuring an analyte by using a camera according to a second embodiment of the present invention.

As illustrated in Fig. 9, in the method of quantitatively measuring an analyte by using a camera according to a second embodiment of the present invention, one side of an analyte kit including an analyte reaction result and an identification code is photographed in the order of a first image and a second image (S910, S930). In this case, the first image denotes a first area image, and the second image denotes a second area image.

After the first image and the second image are photographed, the analyte reaction result is read from the first image (S920).

And also, the identification code is read from the second area (S940).

Accordingly, after the first image and the second image are photographed, a result of reading the analyte reaction result is analyzed using a result of reading of the identification code image, and then an analysis result is generated (S950).

In this case, in order to analyze the result of reading the analyte reaction result, data corresponding to the identification code stored in a storage unit may be used.

And then, the generated reaction result, i.e., effective information about the analyte is output (S960).

In particular, the cases where the identification code is the expire date information, the analyte type information, and the manufacturing lot information may be implemented through the processes of Fig. 8.

While the invention has been particularly shown and described with reference to exemplary embodiments thereof, it will be understood that various changes in form and details may be made therein without departing from the spirit and scope of the following claims.

## Claims

1. A quantitative analyte measurement device for quantitatively measuring an analyte in blood, the quantitative analyte measurement device comprising:
a camera (310) photographing a first virtual region and a second virtual region of an analyte kit (300) in that order, wherein the first virtual region corresponds to an analyte reaction result obtained by reaction of the analyte in blood and the second virtual region corresponds to an identification code (340) of the analyte kit; a read unit (730) reading images of the photographed analyte reaction result and identification code (340) comprising a first read unit (731) for reading a first image of the first virtual region and a second read unit (732) for reading a second image of the second virtual region; wherein
the first image does not include the second image of the second virtual region and the second image does not include the first image of the first virtual region; and
the quantitative analyte measurement device comprises a control unit (750) allowing a result of reading the first image to be processed using a result of reading the second image.

2. The quantitative analyte measurement device of claim 1, wherein the identification code (340) comprises at least one of a bar code, color pattern code, character, or number.

3. The quantitative analyte measurement device of claim 1, wherein the identification code (340) comprises manufacturing lot information of the analyte kit (300), and
the control unit (750) allows the result of reading the image of the analyte reaction result to be calibrated using the manufacturing lot information.

4. The quantitative analyte measurement device of claim 1, wherein the identification code (340) comprises an expire date of the analyte kit (300),
the camera (310) photographs the identification code before the analyte of blood is injected, and
the control unit (750) allows an error message to be output if the expire date of the analyte kit (300) has passed.

5. The quantitative analyte measurement device of claim 1, wherein the identification code (340) comprises analyte type information about types of analytes capable of being analyzed in the analyte kit (300),
the camera (310) photographs the identification code (340) before the analyte of blood is injected, and
the control unit (750) allows an error message to be output if the analyte has the type which cannot be analyzed in the analyte kit (300).

6. A quantitative analyte measurement method of quantitatively measuring an analyte in blood by using a camera (310), the quantitative analyte measurement method comprising:
photographing with the camera (310) a first virtual region and a second virtual region of an analyte kit (300) in that order, wherein the first virtual region corresponds to an analyte reaction result obtained by reaction of the analyte in blood and the second virtual region corresponds to an identification code (340) of the analyte kit (300); reading images of the photographed analyte reaction result and identification, respectively, wherein reading a first image of the first virtual region using a first reading unit and reading a second image of the second virtual region using a second reading unit; wherein
the first image does not include the second image of the second virtual region and the second image does not include the first image of the first virtual region; and
the quantitative analyte measurement method comprises processing a result of reading the first image using a result of reading the second image.

## Patentansprüche

1. Quantitative Analytenmessvorrichtung zur quantitativen Messung eines Analyten in Blut, wobei die quantitative Analytenmessvorrichtung umfasst:
eine Kamera (310), die eine erste virtuelle Region und eine zweite virtuelle Region eines Analyten-Kits (300) in dieser Reihenfolge fotografiert, wobei die erste virtuelle Region einem Analytenreaktionsergebnis entspricht, das durch die Reaktion des Analyten in Blut erhalten wird, und die zweite virtuelle Region einem Identifikationscode (340) des Analyten-Kits entspricht;
eine Leseeinheit (730), die Bilder des fotografierten Analytenreaktionsergebnisses und Identifikationscodes (340) liest, umfassend eine erste Leseeinheit (731) zum Lesen eines ersten Bilds der ersten virtuellen Region und eine zweite Leseeinheit (732) zum Lesen eines zweiten Bilds der zweiten virtuellen Region; wobei
das erste Bild das zweite Bild der zweiten virtuellen Region nicht enthält, und das zweite Bild das erste Bild der ersten virtuellen Region nicht enthält; und
die quantitative Analytenmessvorrichtung eine Steuereinheit (750) umfasst, die es ermöglicht, dass ein Ergebnis des Lesens des ersten Bilds unter Verwendung eines Ergebnisses des Lesens des zweiten Bilds verarbeitet wird.

2. Quantitative Analytenmessvorrichtung nach Anspruch 1,
wobei der Identifikationscode (340) mindestens eines von einem Strichcode, einem Farbmustercode, einem Zeichen oder einer Zahl umfasst.

3. Quantitative Analytenmessvorrichtung nach Anspruch 1,
wobei der Identifikationscode (340) Herstellungslosinformationen des Analyten-Kits (300) umfasst, und
die Steuereinheit (750) ermöglicht, dass das Ergebnis des Lesens des Bilds des Analytenreaktionsergebnisses unter Verwendung der Herstellungslosinformationen kalibriert wird.

4. Quantitative Analytenmessvorrichtung nach Anspruch 1,
wobei der Identifikationscode (340) ein Ablaufdatum des Analyten-Kits (300) umfasst,
die Kamera (310) den Identifikationscode fotografiert, bevor der Analyt von Blut injiziert wird, und
die Steuereinheit (750) ermöglicht, dass eine Fehlernachricht ausgegeben wird, wenn das Ablaufdatum des Analyten-Kits (300) verstrichen ist.

5. Quantitative Analytenmessvorrichtung nach Anspruch 1,
wobei der Identifikationscode (340) Analytentypinformationen über Typen von Analyten umfasst, die in dem Analyten-Kit (300) analysiert werden können,
die Kamera (310) den Identifikationscode (340) fotografiert, bevor der Analyt von Blut injiziert wird, und
die Steuereinheit (750) ermöglicht, dass eine Fehlernachricht ausgegeben wird, wenn der Analyt den Typ aufweist, der in dem Analyten-Kit (300) nicht analysiert werden kann.

6. Quantitatives Analytenmessverfahren zur quantitativen Messung eines Analyten in Blut unter Verwendung einer Kamera (310), wobei das quantitative Analytenmessverfahren umfasst:
Fotografieren mit der Kamera (310) einer ersten virtuellen Region und einer zweiten virtuellen Region eines Analyten-Kits (300) in dieser Reihenfolge, wobei die erste virtuelle Region einem Analytenreaktionsergebnis entspricht, das durch die Reaktion des Analyten in Blut erhalten wird, und die zweite virtuelle Region einem Identifikationscode (340) des Analyten-Kits (300) entspricht;
Lesen von Bildern des fotografierten Analytenreaktionsergebnisses bzw. der Identifikation, wobei
Lesen eines ersten Bilds der ersten virtuellen Region unter Verwendung einer ersten Leseeinheit und Lesen eines zweiten Bilds der zweiten virtuellen Region unter Verwendung einer zweiten Leseeinheit; wobei
das erste Bild das zweite Bild der zweiten virtuellen Region nicht enthält, und das zweite Bild das erste Bild der ersten virtuellen Region nicht enthält; und
das quantitative Analytenmessverfahren das Verarbeiten eines Ergebnisses des Lesens des ersten Bilds unter Verwendung eines Ergebnisses des Lesens des zweiten Bilds umfasst.

## Revendications

1. Dispositif de mesure quantitative d'un analyte permettant de mesurer quantitativement un analyte dans le sang, le dispositif de mesure quantitative d'un analyte comprenant :
un appareil de prise de vues (310) photographiant une première région virtuelle et une seconde région virtuelle d'un kit d'analyte (300) dans cet ordre, la première région virtuelle correspondant à un résultat de réaction d'analyte obtenu par une réaction de l'analyte dans le sang et la seconde région virtuelle correspondant à un code d'identification (340) du kit d'analyte ;
une unité de lecture (730) lisant les images du résultat de la réaction d'analyte photographié et du code d'identification (340) comprenant
une première unité de lecture (731) pour lire une première image de la première région virtuelle et une seconde unité de lecture (732) pour lire une seconde image de la seconde région virtuelle ; dans laquelle
la première image ne comprend pas la seconde image de la seconde région virtuelle et la seconde image ne comprend pas la première image de la première région virtuelle ; et
le dispositif de mesure quantitative d'un analyte comprend une unité de commande (750) permettant qu'un résultat de lecture de la première image soit traité à l'aide d'un résultat de lecture de la seconde image.

2. Dispositif de mesure quantitative d'un analyte selon la revendication 1, dans lequel le code d'identification (340) comprend au moins un parmi un code-barres, un code à motif couleur, un caractère et un nombre.

3. Dispositif de mesure quantitative d'un analyte selon la revendication 1, dans lequel le code d'identification (340) comprend des informations de lot de fabrication du kit d'analyte (300), et
l'unité de commande (750) permet que le résultat de lecture de l'image du résultat de la réaction d'analyte soit étalonné à l'aide des informations de lot de fabrication.

4. Dispositif de mesure quantitative d'un analyte selon la revendication 1, dans lequel le code d'identification (340) comprend une date d'expiration du kit d'analyte (300),
l'appareil de prise de vues (310) photographie le code d'identification avant que l'analyte du sang soit injecté, et
l'unité de commande (750) permet qu'un message d'erreur soit émis si la date d'expiration du kit d'analyte (300) est dépassée.

5. Dispositif de mesure quantitative d'un analyte selon la revendication 1, dans lequel le code d'identification (340) comprend des informations de types d'analyte concernant des types d'analytes susceptibles d'être analysés dans le kit d'analyte (300),
l'appareil de prise de vues (310) photographie le code d'identification (340) avant que l'analyte du sang soit injecté, et
l'unité de commande (750) permet qu'un message d'erreur soit émis si l'analyte est du type qui ne peut pas être analysé dans le kit d'analyte (300).

6. Procédé de mesure quantitative d'un analyte consistant à mesurer quantitativement un analyte dans le sang à l'aide d'un appareil de prise de vues (310), le procédé de mesure quantitative d'un analyte consistant à :
photographier avec l'appareil de prise de vues (310) une première région virtuelle et une seconde région virtuelle d'un kit d'analyte (300) dans cet ordre, la première région virtuelle correspondant à un résultat de réaction d'analyte obtenu par la réaction de l'analyte dans le sang et la seconde région virtuelle correspondant à un code d'identification (340) du kit d'analyte (300) ;
lire des images du résultat de la réaction d'analyte photographié et de l'identification, respectivement,
lire une première image de la première région virtuelle à l'aide d'une première unité de lecture et lire une seconde image de la seconde région virtuelle à l'aide d'une seconde unité de lecture ;
la première image ne comprenant pas la seconde image de la seconde région virtuelle et la seconde image ne comprenant pas la première image de la première région virtuelle ; et
le procédé de mesure quantitative d'un analyte consistant à traiter un résultat de lecture de la première image à l'aide d'un résultat de lecture de la seconde image.
